# FASCICULE DE BREVET EUROPEEN

(11) **EP 0 473 506 B1**
(45) Date de publication et mention de la délivrance du brevet: **13.12.1995**
(21) Numéro de dépôt: 91402316.3
(22) Date de dépôt: 26.08.1991
(51) Int. Cl.: C07C 15/00, C07C 7/00

(54) **Procédé de conversion d'hydrocarbures aliphatiques en hydrocarbures aromatiques**
Verfahren zur Umsetzung von aliphatischen Kohlenwasserstoffen in aromatische Kohlenwasserstoffe
Process for the conversion of aliphatic hydrocarbons in aromatic hydrocarbons

(30) Priorité: 29.08.1990 FR 9010847
(43) Date de publication de la demande: 04.03.1992
(73) Titulaire: INSTITUT FRANCAIS DU PETROLE, 92502 Rueil-Malmaison (FR)
(72) Inventeur: Minkkinen, Ari, F-78860 Saint Nom La Breteche (FR); Renard, Pierre, F-78860 Saint Nom La Breteche (FR); Mank, Larry, F-78630 Orgeval (FR)

(56) Documents cités:
- Aucun document pertinent relevé

## Description

L'invention vise, plus particulièrement, à convertir des hydrocarbures aliphatiques légers, par exemple C2 à C6, particulièrement C3 et/ou C4, en : (1) de l'hydrogène de pureté élevée, et ne renfermant notamment que des traces d'hydrocarbures aromatiques, et (2) des hydrocarbures aromatiques légers seuls ou en mélange (BTX).

On connait divers procédés de conversion d'hydrocarbures aliphatiques C2-C6, dans lesquels l'effluent est un mélange d'hydrogène, d'hydrocarbures aliphatiques légers, notamment de C1 à C5, et d'hydrocarbures aromatiques légers, notamment du benzène, du toluène et/ou du xylène ou leurs mélanges (BTX). Ces procédés sont désignés de plusieurs façons, notamment procédés d'aromatisation ou de déhydrocyclodimérisation. Notamment, dans ce dernier procédé, on convertit des paraffines ou oléfines légères, par exemple C3 et/ou C4, en hydrocarbures aromatiques légers au contact de catalyseurs zéolithiques.

La conversion d'hydrocarbures aliphatiques en hydrocarbures aromatiques est décrite, par exemple dans US 4133743, 4210519, 4233268 et 4172027.

L'aromatisation des hydrocarbures aliphatiques en hydrocarbures aromatiques est aussi décrite, par exemple, dans les brevets français 2634139 et 2634140 et dans les brevets US 3761389 et 4180689. On utilise, par exemple, des catalyseurs du type H-ZSM-5, des aluminosilicates modifiés par ajout de gallium ou de zinc ou des gallosilicates. On opère à une température habituellement comprise entre 500 et 750°C sous une pression de 0,5 à 30 bars et à une vitesse de 0,1 à 5 vol./vol./h.

A la suite de ces procédés, et de manière conventionnelle, l'hydrogène est séparé dans un séparateur haute pression et les hydrocarbures sont séparés dans un train de colonnes de distillation.

On a aussi proposé l'emploi de membranes perm-sélectives pour séparer l'hydrogène des hydrocarbures, par exemple dans US 4180388, 4398926 et 4654047. L'emploi d'une membrane perm-sélective et de colonnes de fractionnement est décrit dans US 4548619. Dans ce dernier brevet, l'effluent d'une unité de déhydrocyclodimérisation est d'abord fractionné sommairement ; la fraction liquide est distillée pour recueillir les BTX et la fraction gazeuse est comprimée puis lavée par des hydrocarbures aromatiques ou par des hydrocarbures paraffiniques C7 - C10 d'origine externe.

La présente invention concerne un procédé d'aromatisation d'hydrocarbures aliphatiques C3 et/ou C4 comportant un fractionnement du mélange gazeux obtenu renfermant de l'hydrogène, des hydrocarbures aliphatiques légers et des hydrocarbures aromatiques, économe en énergie, en particulier un procédé dans lequel les besoins en énergie pour le fractionnement des produits sont réduits ; et également un procédé dans lequel l'hydrogène obtenu est sensiblement libre d'hydrocarbures aromatiques ; un procédé aussi qui permet d'utiliser des membranes sensibles aux hydrocarbures aromatiques, en raison de la quasi-absence de ceux-ci dans le gaz soumis à perméation. Dans le procédé de l'invention, la cristallisation indésirable d'hydrocarbures aromatiques est évitée.

Ce procédé est applicable en particulier au propane et au butane, seuls ou en mélange, avec éventuellement des hydrocarbures aliphatiques C2 ou C5⁺.

Dans le procédé de l'invention, on convertit une première portion de la charge hydrocarbonée fraîche, renfermant au moins un hydrocarbure aliphatique C3 et/ou C4, au contact d'un catalyseur d'aromatisation, dans les conditions de l'aromatisation. L'effluent gazeux de la zone de conversion d'hydrocarbures, qui renferme de l'hydrogène, des hydrocarbures aliphatiques légers et des hydrocarbures aromatiques légers, est ensuite refroidi à une température permettant la condensation d'une partie des hydrocarbures. On sépare une première fraction gazeuse non condensée relativement pauvre en aromatiques et une première fraction liquide relativement riche en aromatiques.

Dans une forme de réalisation préférée, la charge hydrocarbonée renfermant au moins un hydrocarbure C3 et/ou C4 est à l'état liquide et la première portion de cette charge est détendue et soumise à un échange de chaleur indirect avec l'effluent du réacteur de conversion, ce qui provoque la vaporisation de ladite première portion et le refroidissement et la condensation partielle dudit effluent. Ceci contribue efficacement à l'économie du procédé.

On met en contact la première fraction gazeuse avec une seconde portion de la charge hydrocarbonée fraîche à l'état liquide dans une zone de contact, dans des conditons assurant à la fois la vaporisation d'au moins une fraction, par exemple au moins 50 % (de préférence 60-95%), de ladite seconde portion de la charge hydrocarbonée fraîche, et la condensation d'au moins une partie des hydrocarbures aromatiques de la première fraction gazeuse, ladite condensation étant provoquée, au moins en partie, par le refroidissement dû à ladite vaporisation des hydrocarbures aliphatiques, et on sépare une seconde fraction gazeuse d'une seconde fraction liquide comprenant des hydrocarbures aliphatiques et des hydrocarbures aromatiques.

On traite si nécessaire la seconde fraction gazeuse pour l'amener au-dessus de son point de rosée, on la fait circuler au contact d'un moins une membrane perméable à l'hydrogène et on recueille une fraction gazeuse enrichie en hydrogène et une troisième fraction gazeuse appauvrie en hydrogène. On refroidit la troisième fraction gazeuse de manière à la condenser partiellement et on recueille une quatrième fraction gazeuse, riche en méthane, qui peut constituer un "fuel-gas" (gaz combustible), et une troisième fraction liquide renfermant au moins un hydrocarbure C3 et/ou C4.

On soumet les première, seconde et troisième fractions liquides à une distillation, ensemble ou séparément, en une ou plusieurs étapes, et on recueille en tête au moins une cinquième fraction gazeuse renfermant au moins un hydrocarbure C3 et/ou C4 et en queue au moins une quatrième fraction liquide qui constitue une fraction d'hydrocarbures aromatiques recherchée. Au moins une partie des hydrocarbures de la cinquième fraction gazeuse sont condensés et le condensat obtenu est détendu et mis au contact d'échange indirect de chaleur avec la troisième fraction gazeuse. La vaporisation de ce condensat permet de refroidir la troisième fraction gazeuse et contribue en large partie à sa liquéfaction, ce qui constitue un avantage essentiel du procédé. Le condensat ainsi vaporisé est finalement envoyé à la zone de conversion d'hydrocarbures pour constituer une partie de la charge hydrocarbonée de cette zone, qui, comme indiqué ci-dessus, reçoit aussi une première portion de la charge hydrocarbonée fraîche.

Dans un mode de réalisation préféré, la première portion de la charge hydrocarbonée, qui est envoyée relativement directement au réacteur de conversion, et la seonde portion de la charge hydrocarbonée, qui est envoyée à la zone de contact avec la première fraction gazeuse, mais qui reviendra au moins en partie au réacteur de conversion après être passée dans le train de séparateurs décrit ci-dessus, représentent respectivement 50 à 95 % et 5 à 50 % de la charge hydrocarbonée totale.

Les deux fractions de la charge hydrocarbonée n'ont pas nécessairement la même composition ou la même origine. Ainsi, la première portion peut être constituée d'hydrocarbures relativement plus légers que ceux de la seconde portion, par exemple en majorité C3 ou C3-C4 pour la première portion et C5 ou C4-C5 pour la seconde portion.

On peut avantageusement vaporiser au moins une partie de la première portion au contact de la troisième fraction gazeuse pour refroidir celle-ci.

Le réacteur de conversion d'hydrocarbures peut être, par exemple, un réacteur d'aromatisation d'hydrocarbures légers C2-C5, particulièrement C3 et/ou C4, utilisant une zéolithe comme catalyseur, notamment une zéolithe décrite dans le brevet français 2634139 ou 2634140.

La pression en sortie de réacteur est, par exemple, de 1,5 à 10 bars, couramment 2-5 bars. Si la température est élevée, on abaisse celle-ci, par exemple vers 10-60°C, de préférence 30-50°C, de manière à condenser une partie de l'effluent gazeux du réacteur et à recueillir au moins une partie des hydrocarbures aromatiques. Si on le désire, on peut modifier la pression pour favoriser la condensation des hydrocarbures aromatiques.

La première fraction gazeuse peut être ensuite comprimée jusqu'à, par exemple, 15-40 bars, de préférence 20-30 bars, et si nécessaire refroidie, pour amener sa température vers -10 à +55°C, de préférence 5-35°C, et condenser au moins une seconde fraction liquide, renfermant des aromatiques. On sépare cette fraction liquide de la seconde fraction gazeuse sous la pression précitée. Au lieu d'un seul étage de compression suivi de refroidissement et de fractionnement, on peut utiliser plusieurs étages de compression, suivis chacun d'une condensation partielle et d'un fractionnement.

Elle est alors mise en contact avec la seconde portion de la charge hydrocarbonée fraîche à l'état liquide, renfermant au moins un hydrocarbure C3 et/ou C4. La vaporisation d'au moins 50 % des hydrocarbures de cette seconde portion provoque la condensation d'au moins une partie des hydrocarbures aromatiques résiduels. La température est, par exemple, entre - 10 et + 40°C, de préférence entre 5 et 35°C. En tête la température est par exemple entre - 10 et + 30°C, de préférence entre 0 et 20°C. En fond elle est par exemple de 5 à 40°C, de préférence de 10 à 25°C ; la pression peut être 15-40 bars, de préférence 20-30 bars. Exprimée en poids, la quantité de seconde portion de charge hydrocarbonée à l'état liquide peut représenter, par exemple, 5 à 35 %, de préférence 10 à 25 %, de la quantité de première fraction gazeuse.

Dans certains cas, notamment quand la première fraction gazeuse est très riche en C3 -C4, il peut être avantageux que la seconde portion de charge hydrocarbonée à l'état liquide contienne également une certaine proportion d'hydrocarbures non-aromatiques C5 plus.

Si nécessaire le courant gazeux résultant (seconde fraction gazeuse) est alors amené au-dessus de son point de rosée, par exemple par chauffage ou par dilution avec un gaz sec, mais de préférence par compression supplémentaire assurant une surchauffe (un incrément de 2 à 7 bars est généralement suffisant). Il est ensuite mis au contact d'au moins une membrane de perméation sélective, en un ou plusieurs étages. La surchauffe est de préférence telle qu'aucune condensation ne se produit lors du soutirage d'hydrogène dans la membrane, ce qui est avantageux si la membrane est sensible à la présence des hydrocarbures liquides.

Pour la perméation, on peut se référer à l'un des brevets précités, et on opérera de préférence en plusieurs étages avec recompression du gaz entre les étages. La membrane de perméation peut être une membrane du commerce ou de l'art antérieur et ne sera donc pas décrite en détail. Les conditions opératoires dépendent de la membrane, par exemple vers 30-150°C sous 20-40 bars avec les membranes usuelles. A la sortie de l'étage de perméation la fraction de gaz appauvrie en hydrogène (troisième fraction gazeuse) et qui renferme usuellement des hydrocarbures C1-C5 est soumise à refroidissement pour condenser une phase liquide (troisième fraction liquide) renfermant des hydrocarbures C3-C5. Le refroidissement peut utiliser, pour partie, des courants relativement froids du procédé, par exemple le courant de la quatrième fraction gazeuse, et, pour partie, des courants de gaz liquéfié, par exemple un courant d'éthane, de propane ou de butane liquide. De préférence on utilise un courant C3-C4 liquéfié comme indiqué plus loin.

La distillation des fractions liquides (première, seconde, troisième) peut être réalisée séparément ou après mélange de 2 ou plus fractions.

De préférence on distille ensemble ces 3 fractions.

Selon un mode préféré de réalisation, à la sortie de la zone de contact, la seconde fraction gazeuse est comprimée avant passage dans la zone de perméation. Le compresseur peut alors être en ligne avec les compresseurs des étages précédents. Un avantage important de cette compression et de son effet thermique est de placer le mélange gazeux au-dessus du point de rosée des hydrocarbures privés de l'hydrogène qui seront obtenus à la sortie de la zone de perméation.

Selon une autre forme de réalisation, le compresseur pour la seconde fraction gazeuse reçoit l'énergie, de préférence l'énergie mécanique, produite par un détendeur placé sur le circuit de la quatrième fraction gazeuse. Dans ce cas on utilise de préférence respectivement un turbocompresseur et un turbodétendeur. Selon une variante, il n'y a pas d'étage de compression pour la seconde fraction gazeuse mais un simple chauffage et le turbodétendeur transmet son énergie à un ou plusieurs étages compresseurs de la première fraction gazeuse.

Selon une autre forme de réalisation la cinquième fraction gazeuse est soumise à une condensation partielle : une partie au moins du condensat riche en butanes et pentanes est renvoyée comme reflux à la colonne où l'on a séparé ladite cinquième fraction gazeuse de ladite quatrième fraction liquide ; la partie non-condensée est renvoyée au réacteur de déhydrocyclodimérisation.

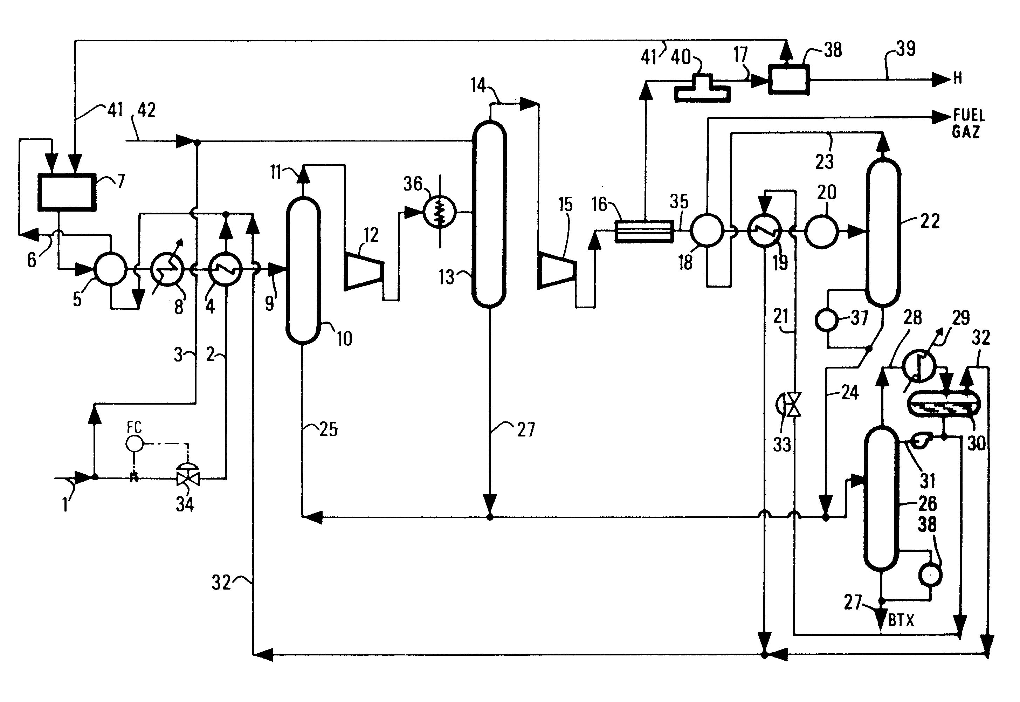

La figure illustre un mode non limitatif de mise en oeuvre de l'invention.

Dans ce qui suit les échangeurs sont tous à contact indirect, à travers une paroi.

La charge liquide fraîche (1) d'hydrocarbures C3-C4, pouvant renfermer en outre des hydrocarbures C2 et/ou C5⁺, est divisée en deux courants (2) et (3). Le courant (2) est détendu (34) et passe dans l'échangeur (4), puis dans l'échangeur (5) pour parvenir par la ligne (6) au réacteur (7) où il est mis en contact avec un catalyseur d'aromatisation, par exemple un aluminosilicate additionné de gallium.

L'effluent du réacteur (7) passe dans l'échangeur (5), puis si nécessaire dans l'échangeur (8). Il passe ensuite dans l'échangeur (4) où il cède de la chaleur nécessaire à la vaporisation de la première portion de la charge hydrocarbonée et parvient par la ligne (9) au ballon (10). L'effluent gazeux de tête (11) est comprimé (12) puis refroidi (36) et mis au contact de la portion de charge liquide hydrocarbonée (3) dans le contacteur (13). On recueille une fraction liquide fortement aromatique (25).

La phase gazeuse (14) subit une surchauffe, par exemple celle qui résulte du passage dans le compresseur (15), puis passe dans l'unité de perméation (16).

De l'hydrogène purifié sort par la ligne (17). Le gaz résiduel (35) subit un refroidissement, par exemple dans les échangeurs (18, 19 et 20), par un courant de gaz froid (19), par un courant de C3-C4 liquide détendu à basse température, par exemple - 30 à - 40°C (21), et par un refroidisseur auxiliaire, si nécessaire (20). Une liquéfaction partielle se produit et l'on recueille en tête de la colonne (22) un gaz riche en méthane, par la ligne (23) et un courant liquide (24) au bas de la colonne (22). Dans le cas considéré, le courant liquide (25) soutiré du contacteur (10) est envoyé à la colonne (26), qui reçoit aussi le courant liquide (27) soutiré du ballon (13) et le courant (24) soutiré de la colonne (22). Un mélange riche en hydrocarbures aromatiques est recueilli par la ligne (27). En tête les vapeurs (28), riches en hydrocarbures (C3-C4) sont refroidies et partiellement condensées (29). Dans le séparateur (30) on recueille une phase liquide, par exemple C3-C4-C5, qui est renvoyée au réacteur (7) après passage dans le détendeur (33), la ligne (21), l'échangeur (19), l'échangeur (5) et la ligne (6). Dans l'échangeur (19) la chaleur de vaporisation sert à refroidir la troisième fraction gazeuse. On peut assurer un reflux par la ligne (31). S'il reste une phase gazeuse (32), elle peut être renvoyée au réacteur de déhydrocyclodimérisation ou purgée.

Les rebouilleurs des colonnes (22) et (26) sont désignés respectivement par (37) et (38).

Dans une forme de réalisation préférée, permettant d'obtenir de l'hydrogène de haute pureté et d'accroître le rendement, on soumet le courant d'hydrogène provenant de la membrane de perméation (17) après passage éventuel dans un compresseur (40) à une séparation par adsorption, par exemple du type PSA (38). On recueille l'hydrogène purifié (39) et les hydrocarbure rejetés peuvent être recyclés (ligne 41).

On peut aussi ajouter des hydrocarbures C5 ou C5-C6 (ligne 42) au liquide de la ligne (3) lorsque celui-ci est constitué presque exclusivement d'hydrocarbures C3 ou C3-C4.

A titre d'exemple, on traite 77894 parties (pds)/heure d'une charge renfermant (en poids) 3,5 % d'hydrogène, 10,9 % de C1-C2, 36,2 % de C3-C5, et 49,4 % de BTX. Après séparation dans (10), compression dans (12) jusqu'à 22 bars et refroidissement à 50°C dans (36), la colonne (13) reçoit 49415 parties (pds)/heure d'un courant 7,0 % pds de BTX. Par la ligne (3), on envoie 12025 parties (pds)/h de coupe C3C4C5. La température est de 33°C en tête et 47°C en fond. Le courant de tête ne renferme que 0,4 % pds de BTX, la concentration de BTX dans le courant de fond étant de 67 % pds. Environ 42 % des C3C4C5 sortent en fond et 58 % en tête. Après surcompression de 5 bars (P totale = 27 bars), qui augmente le degré de surchauffe du mélange gazeux de 20°C, on enlève plus de 80 % de l'hydrogène au contact des membranes. Après distillation des courants liquides (24, 25, 27), on établit le bilan : on a recueilli 2770 parties (pds)/h d'hydrogène d'une pureté de 97,5 %, 16639 parties (pds)/h de gaz combustible, 40600 parties (pds)/h de coupe C3-C4-C5 qui sont recyclées et 29910 parties (pds)/h de coupe riche en BTX.

## Revendications

1. Procédé de conversion d'hydrocarbures aliphatiques en hydrocarbures aromatiques, dans lequel on convertit une charge hydrocarbonée fraîche d'hydrocarbures aliphatiques renfermant au moins un hydrocarbure à 3 ou 4 atomes de carbone au contact d'un catalyseur d'aromatisation et on fractionne les produits obtenus par perméation de l'hydrogène et distillation des hydrocarbures, caractérisé en ce que :
(a) on convertit une première portion de la charge hydrocarbonée fraîche au contact d'un catalyseur d'aromatisation, dans des conditions d'aromatisation,
b) on refroidit l'effluent de (a) pour condenser une partie des hydrocarbures, et on sépare une première fraction gazeuse non condensée, relativement pauvre en aromatiques et une première fraction liquide condensée, relativement riche en aromatiques,
c) on met en contact direct la première fraction gazeuse avec une seconde portion de la charge hydrocarbonée fraîche à l'état liquide, dans des conditions assurant à la fois la vaporisation d'au moins une fraction de ladite seconde portion de la charge hydrocarbonée fraîche et la condensation d'au moins une partie des hydrocarbures aromatiques de la première fraction gazeuse, et on sépare une seconde fraction gazeuse d'une seconde fraction liquide,
d) on fait circuler la seconde fraction gazeuse au contact d'au moins une membrane perméable à l'hydrogène, et on recueille une fraction gazeuse enrichie en hydrogène et une troisième fraction gazeuse appauvrie en hydrogène,
e) on refroidit la troisième fraction gazeuse de manière à la condenser en partie et on recueille une quatrième fraction gazeuse, enrichie en méthane, et une troisième fraction liquide renfermant au moins un hydrocarbure C3 et/ou C4,
f) on distille, ensemble ou séparément, les première, seconde et troisième fractions liquides et on recueille au moins une cinquième fraction gazeuse, renfermant au moins un hydrocarbure C3 et/ou C4, et au moins une quatrième fraction liquide contenant des hydrocarbures aromatiques,
g) on condense au moins une partie des hydrocarbures de la cinquième fraction gazeuse, on détend le condensat ainsi obtenu et on le met en contact d'échange indirect de chaleur avec la troisième fraction gazeuse pour provoquer sa vaporisation au moins partielle, et
h) on envoie au moins une partie du condensat vaporisé, obtenu à l'étape (g), à la conversion de l'étape (a) pour y être convertie en mélange avec la première portion de la charge hydrocarbonée fraîche.

2. Procédé selon la revendication 1 caractérisé en ce que l'étape (b) est réalisée à 10-60°C sous 1,5 -10 bars et l'étape (c) est réalisée à - 10°C jusqu'à + 40°C sous 1,5-10 bars.

3. Procédé selon la revendication 2, caractérisé en ce que l'étape (b) est réalisée à 30-50°C sous 1,5 - 10 bars, l'étape (c) est réalisée à 5-35°C sous 1,5 -10 bars et l'étape (d) est réalisée à 30-150°C sous 20-40 bars.

4. Procédé selon l'une des revendications 1 à 3 caractérisé en ce qu'on élève la température de la seconde fraction gazeuse pour l'amener au-dessus de son point de rosée, de telle manière qu'il ne se produise pas de condensation d'hydrocarbure au contact de la membrane.

5. Procédé selon l'une des revendications 1 à 4, caractérisé en ce que dans l'étape (c), on vaporise au moins 50 % des hydrocarbures aliphatiques de la seconde portion de la charge hydrocarbonée fraîche.

6. Procédé selon l'une des revendications 1 à 5, caractérisé en ce que, dans l'étape (c), on vaporise 60 à 95 % des hydrocarbures aliphatiques de la seconde portion de la charge hydrocarbonée fraîche.

7. Procédé selon l'une des revendications 1 à 6, caractérisé en ce que l'on distille ensemble les première, seconde et troisième fractions liquides.

8. Procédé selon l'une des revendications 1 à 7, caractérisé en ce que la charge hydrocarbonée fraîche est initialement à l'état liquide et on détend la première portion de ladite charge hydrocarbonée puis on la vaporise avant de l'envoyer à la conversion de l'étape (a), la chaleur de vaporisation étant prise par échange indirect avec l'effluent de ladite étape (a).

9. Procédé selon l'une des revendications 1 à 8, caractérisé en ce que les première et seconde portions de la charge hydrocarbonée représentent respectivement 50 - 95 % et 5 - 50 % de cette charge.

10. Procédé selon l'une des revendications 1 à 9, caractérisé en ce que la fraction gazeuse enrichie en hydrogène recueillie à l'étape (d) est purifiée par adsorption, de manière à accroître la pureté de l'hydrogène, et les hydrocarbures rejetés sont recyclés à la conversion de l'étape (a).

## Claims

1. A process for the conversion of aliphatic hydrocarbons to aromatic hydrocarbons, in which a fresh hydrocarbon feed of aliphatic hydrocarbons comprising at least one hydrocarbon containing 3 or 4 carbon atoms is converted in contact with an aromatization catalyst and the products obtained are fractionated by hydrogen permeation and distillation of the hydrocarbons, characterised in that:
(a) a first portion of the fresh hydrocarbon feed is converted in contact with an aromatization catalyst under aromatization conditions,
(b) the effluent from (a) is cooled to condense a portion of the hydrocarbons, and an uncondensed first gaseous fraction which is relatively depleted in aromatics and a first condensed liquid fraction which is relatively rich in aromatics are separated,
(c) the first gaseous fraction is brought into direct contact with a second portion of the fresh hydrocarbon feed in the liquid state, under conditions such that at least a fraction of said second portion of fresh hydrocarbon feed vaporizes and at least a portion of the aromatic hydrocarbons in the first gaseous fraction condenses, and a second gaseous fraction is separated from a second liquid fraction,
(d) the second gaseous fraction is circulated in contact with at least one membrane which is permeable to hydrogen, and a gaseous fraction which is enriched in hydrogen and a third gaseous fraction which is depleted in hydrogen are recovered,
(e) the third gaseous fraction is cooled to partially condense it and a fourth gaseous fraction, which is enriched in methane, and a third liquid fraction containing at least one C3 and/or C4 hydrocarbon are recovered,
(f) the first, second and third liquid fractions are distilled, either together or separately, and at least a fifth gaseous fraction comprising at least one C3 and/or C4 hydrocarbon, and at least a fourth liquid fraction containing aromatic hydrocarbons are recovered,
(g) at least a portion of the hydrocarbons in the fifth gaseous fraction is condensed, the condensate obtained is decompressed and placed in indirect heat exchange contact with the third gaseous fraction to cause at least partial vaporization of the latter, and
(h) at least a portion of the vaporized condensate obtained from step (g) is sent to conversion step (a) for conversion as a mixture with the first portion of the fresh hydrocarbon feed.

2. A process according to claim 1, characterised in that step (b) is carried out at 10-60°C at 1.5-10 bars and step (c) is carried out at -10°C to +40°C at 1.5-10 bars.

3. A process according to claim 2, characterised in that step (b) is carried out at 30-50°C at 1.5-10 bars, step (c) is carried out at 5-35°C at 1.5-10 bars and step (d) is carried out at 30-150°C at 20-40 bars.

4. A process according to any one of claims 1 to 3, characterised in that the temperature of the second gaseous fraction is raised above its dew point so that the hydrocarbon in contact with the membrane does not condense.

5. A process according to any one of claims 1 to 4, characterised in that in step (c), at least 50% of the aliphatic hydrocarbons of the second portion of fresh hydrocarbon feed is vaporized.

6. A process according to any one of claims 1 to 5, characterized in that in step (c), 60% to 95% of the aliphatic hydrocarbons of the second portion of fresh hydrocarbon feed is vaporized.

7. A process according to any one of claims 1 to 6, characterised in that the first, second and third liquid fractions are distilled together.

8. A process according to any one of claims 1 to 7, characterised in that the fresh hydrocarbon feed is initially in the liquid state and the first portion of said hydrocarbon feed is decompressed then vaporized before sending it to conversion step (a), the heat of evaporation being taken by indirect exchange with the effluent from said step (a).

9. A process according to any one of claims 1 to 8, characterised in that the first and second portions of the hydrocarbon feed respectively represent 50-95% and 5-50% of said feed.

10. A process according to any one of claims 1 to 9, characterised in that the hydrogen-enriched gaseous fraction recovered from step (d) is purified by adsorption, to increase the purity of the hydrogen, and the rejected hydrocarbons are recycled to conversion step (a).

## Patentansprüche

1. Verfahren zur Umwandlung von aliphatischen Kohlenwasserstoffen in aromatische Kohlenwasserstoffe, bei dem man eine frische kohlenwasserstoffhaltige Charge aus aliphatischen Kohlenwasserstoffen, die wenigstens einen Kohlenwasserstoff mit 3 oder 4 Kohlenstoffatomen enthalten, in Kontakt mit einem Aromatisierungskatalysator umwandelt und man die erhaltenen Produkte durch Permeation des Wasserstoffs und Destillation der Kohlenwasserstoffe fraktioniert, dadurch gekennzeichnet, daß:
a) man einen ersten Teil der frischen kohlenwasserstoffhaltigen Charge in Kontakt mit einem Aromatisierungskatalysator unter Aromatisierungsbedingungen umwandelt,
b) man den Abstrom aus (a) zum Kondensieren eines Teils der Kohlenwasserstoffe kühlt und man eine erste gasförmige nicht kondensierte Fraktion, die relativ arm an Aromaten ist und eine erste flüssige kondensierte Fraktion, die relativ reich an Aromaten ist, abtrennt,
c) man die erste gasförmige Fraktion in direkten Kontakt mit einem zweiten Teil der frischen kohlenwasserstoffhaltigen Charge im flüssigen Zustand unter Bedingungen bringt, die gleichzeitig die Verdampfung wenigstens einer Fraktion dieses zweiten Teils der frischen kohlenwasserstoffhaltigen Charge und die Kondensation wenigstens eines Teils der aromatischen Kohlenwasserstoffe der gasförmigen ersten Fraktion sicherstellt und man eine zweite gasförmige Fraktion von einer zweiten flüssigen Fraktion trennt,
d) man die zweite gasförmige Fraktion in Kontakt mit wenigstens einer für Wasserstoff permeablen Membran zirkulieren läßt und man eine an Wasserstoff angereicherte gasförmige Fraktion und eine an Wasserstoff veramte dritte Fraktion sammelt,
c) man die dritte gasförmige Fraktion derart kühlt, daß sie zum Teil kondensiert und man eine vierte gasförmige an Methan angereicherte Fraktion sowie eine dritte flüssige Fraktion sammelt, die wenigstens einen C3- und/oder C4-Kohlenwasserstoff umfaßt,
f) man gemeinsam oder getrennt die ersten, zweiten und dritten flüssigen Fraktionen destilliert und wenigstens eine fünfte gasförmige Fraktion, die wenigstens einen C3- und/oder C4-Kohlenwasserstoff umfaßt und wenigstens eine vierte flüssige aromatische Kohlenwasserstoffe enthaltende Fraktion sammelt,
g) man wenigstens einen Teil der Kohlenwasserstoffe der fünften gasförmigen Fraktion kondensiert, man das so erhaltene Kondensat entspannt und es in indirektem Wärmeaustausch mit der dritten gasförmigen Fraktion, um seine wenigstens partielle Verdampfung hervorzurufen, kontaktiert, und
h) man wenigstens einen Teil des bei der Stufe (g) erhaltenen verdampften Kondensats zur Umwandlung der Stufe (a) schickt, um in dieser im Gemisch mit dem ersten Teil der frischen kohlenwasserstoffhaltigen Charge umgewandelt zu werden.

2. Verfahren nach Anspruch 1, dadurch gekennzeichnet, daß die Stufe (b) bei 10-60°C unter 1,5-10 bar durchgeführt wird und die Stufe (c) bei -10°C bis +40°C unter 1,5-10 bar durchgeführt wird.

3. Verfahren nach Anspruch 2, dadurch gekennzeichnet, daß die Stufe (b) bei 30-50°C unter 1,5-10 bar, die Stufe (c) bei 5-35°C unter 1,5-10 bar und die Stufe (d) bei 30-150°C unter 20-40 bar durchgeführt wird.

4. Verfahren nach einem der Ansprüche 1 bis 3, dadurch gekennzeichnet, daß die Temperatur der zweiten gasförmigen Fraktion erhöht wird, um sie über ihren Taupunkt derart zu führen, daß sich keine Kohlenwasserstoffkondensation in Kontakt mit der Membran einstellt.

5. Verfahren nach einem der Ansprüche 1 bis 4, dadurch gekennzeichnet, daß in der Stufe (c) man wenigstens 50 % der aliphatischen Kohlenwasserstoffe des zweiten Teils der frischen kohlenwasserstoffhaltigen Charge verdampft.

6. Verfahren nach einem der Ansprüche 1 bis 5, dadurch gekennzeichnet, daß man in der Stufe (c) 60 bis 95 % der aliphatischen Kohlenwasserstoffe des zweiten Teils der frischen kohlenwasserstoffhaltigen Charge verdampft.

7. Verfahren nach einem der Ansprüche 1 bis 6, dadurch gekennzeichnet, daß man gemeinsam die ersten, zweiten und dritten flüssigen Fraktionen destilliert.

8. Verfahren nach einem der Ansprüche 1 bis 7, dadurch gekennzeichnet, daß die frische kohlenwasserstoffhaltige Charge zunächst im flüssigen Zustand vorliegt und man den ersten Teil dieser kohlenwasserstoffhaltigen Charge entspannt und sie dann, bevor sie zur Umwandlung der Stufe (a) geschickt wird, verdampft, wobei die Verdampfungswärme durch indirekten Austausch mit dem Abstrom der Stufe (a) gewonnen wird.

9. Verfahren nach einem der Ansprüche 1 bis 8, dadurch gekennzeichnet, daß die ersten und zweiten Teile der kohlenwasserstoffhaltigen Charge jeweils 50 - 95 % und 5 - 50 % dieser Charge darstellen.

10. Verfahren nach einem der Ansprüche 1 bis 9, dadurch gekennzeichnet, daß die an Wasserstoffen angereicherte Endstufe (d) gesammelte Fraktion durch Adsorption derart gereinigt wird, daß die Reinheit des Wasserstoffs gesteigert wird, und die zurückgewiesenen Kohlenwasserstoffe zur Umwandlung der Stufe (a) rezykliert werden.
